# EUROPEAN PATENT SPECIFICATION

(11) **EP 3 020 725 B1**
(45) Date of publication and mention of the grant of the patent: **26.12.2018**
(21) Application number: 15193522.8
(22) Date of filing: 06.11.2015
(51) Int. Cl.: C07K 14/435

(54) **METHOD FOR PRODUCING HYPO-METALLATED REDOX-ACTIVE METALLOTHIONEIN PROTEIN AND PHARMACEUTICAL COMPOSITION CONTAINING THE SAME**
VERFAHREN ZUR HERSTELLUNG HYPOMETALLISIERTEM, REDOXAKTIVEM METALLOTHIONEIN-PROTEIN UND PHARMAZEUTISCHE ZUSAMMENSETZUNG DAMIT
PROCÉDÉ DE PRODUCTION DE PROTÉINE DE MÉTALLOTHIONÉINE À RÉACTION REDOX HYPO-METALLÉE ET COMPOSITION PHARMACEUTIQUE LA CONTENANT

(30) Priority: 11.11.2014 TW 103139135
(43) Date of publication of application: 18.05.2016
(73) Proprietor: Melcher, Christoph, Taipei City 114 (TW); Henry, Tony Raoul, Taipei City 110 (TW); Kuo, Chun-Hung, Taipei City 100 (TW)
(72) Inventor: Melcher, Christoph, 114 Taipei City (TW)
(74) Representative: Lang, Christian

(56) References cited:
- WO-A2-2007/130575
- STEPHEN G. BELL ET AL: "The Metallothionein/Thionein System: An Oxidoreductive Metabolic Zinc Link", CHEMBIOCHEM - A EUROPEAN JOURNAL OF CHEMICAL BIOLOGY., vol. 10, no. 1, 5 January 2009 (2009-01-05), pages 55-62, XP055256375, DE ISSN: 1439-4227, DOI: 10.1002/cbic.200800511
- THANH T. NGU ET AL: "Arsenic transfer between metallothionein proteins at physiological pH", BIOCHEMICAL AND BIOPHYSICAL RESEARCH COMMUNICATIONS, vol. 401, no. 1, 1 October 2010 (2010-10-01), pages 69-74, XP055256234, US ISSN: 0006-291X, DOI: 10.1016/j.bbrc.2010.09.010
- HAJO HAASE ET AL: "Partial oxidation and oxidative polymerization of metallothionein", ELECTROPHORESIS, vol. 29, no. 20, 1 November 2008 (2008-11-01), pages 4169-4176, XP055256384, DE ISSN: 0173-0835, DOI: 10.1002/elps.200700922
- HAASE H ET AL: "A differential assay for the reduced and oxidized states of metallothionein and thionein", ANALYTICAL BIOCHEMISTRY, ACADEMIC PRESS INC, NEW YORK, vol. 333, no. 1, 1 October 2004 (2004-10-01), pages 19-26, XP027195457, ISSN: 0003-2697 [retrieved on 2004-09-09]
- J. Jeong ET AL: "Novel Oxidative Modifications in Redox-Active Cysteine Residues", Molecular & Cellular Proteomics, vol. 10, no. 3, 1 March 2011 (2011-03-01), pages M110.000513-M110.00051, XP055369940, US ISSN: 1535-9476, DOI: 10.1074/mcp.M110.000513
- Amanda Yarnell: "Cysteine Oxidation | Science & Technology | Chemical & Engineering News", , 5 October 2009 (2009-10-05), XP055369952, Retrieved from the Internet: URL:https://pubs.acs.org/cen/science/87/87 40sci1.html [retrieved on 2017-05-08]
- Anonymous: "Chemistry of Cystine", , 8 May 2017 (2017-05-08), XP055369955, Retrieved from the Internet: URL:https://employees.csbsju.edu/hjakubows ki/classes/ch331/protstructure/PS_2A7_Cyst ine_Chem.html [retrieved on 2017-05-08]
- Anonymous: "Reducing Agents for Protein Disulfides | Thermo Fisher Scientific", , 8 May 2017 (2017-05-08), XP055369957, Retrieved from the Internet: URL:https://www.thermofisher.com/nl/en/hom e/life-science/protein-biology/protein-lab eling-crosslinking/protein-modification/re ducing-agents-protein-disulfides.html# [retrieved on 2017-05-08]

## Description

### BACKGROUND OF THE INVENTION

### 1. FIELD OF THE INVENTION

The present invention relates to production of hypo-metallated redox-active metallothionein (MT)proteins, particularly to production of MT proteins with defined numbers of MT cysteinyl binding sites occupied with physiologically relevant metal ions.

### 2. DESCRIPTION OF THE RELATED ART

Oxidative stress is a harmful form of chemical stress constantly occurring in the human body, mainly at the site of intracellular energy conversion, the mitochondria. The leakage of electrons from the mitochondrial electron transport chain is seen as the major cause for intracellular generation of free radicals such as ROS (reactive oxygen species), RNS (reactive nitrogen species), and others. External stimuli like environmental chemicals, radiation, virus infections, as well as malnutrition and lifestyle, contribute to generation of free radicals, leading to internal oxidative stress. Free radicals such as ROS orRNS, display a considerable degree of chemical reactivity due to their comprising of unpaired electrons (Cadenas et al., 2000), and can cause a variety of intracellular damage like DNA oxidation, lipid peroxidation, enzyme inactivation, RNA oxidation and others (Swindell, 2011). If unchecked, such oxidative stress and subsequent molecular damage can manifest in an array of medical conditions (Swindell, 2011). For example, oxidative stress is now seen as the underlying cause of metabolic disorders including but not limited to diabetes, hyperlipidemia and obesity, age-related neurological disorders including but not limited to Parkinson's Disease and Alzheimer's Disease, as well as various forms of cancer (Swindell, 2011; Chimienti, 2013; Furukawa et al., 2004; Özcan et al., 2004; Miyazaki et al., 2008; Vasto et al., 2008; Moreli et al., 2014).

The generation of free radicals is tightly linked with the intracellular redox state, which in turn has to be maintained within strict physiological limits, for normal body function (Jiang et al., 1998). Scavenging and inactivation of free radicals is done by physiological antioxidants like glutathione (GSH), vitamins C and E, lipoic acid, flavonoids and other redox-active components (Aquilano et al., 2014). Redox-activity of such protective antioxidants, again, is tightly linked to the redox state of the cell, and the regeneration of their antioxidant capacity, and in turn the balancing of intracellular redox potential, is done via complex interplay of couples of redox active metal ions, enzymes and substrates, and others (Jiang et al., 1998; Aquilano et al., 2014). Overloading of intrinsic antioxidant protection mechanisms with oxidative stressors, and thus dis-balancing the intracellular redox potential, will inevitably lead to damage and malfunction of intracellular components, manifesting in cellular, tissue, organ and ultimately systemic breakdown.

Of central role in the human body, as redox-active, radical-scavenging and metal ion-binding proteins, linking antioxidant activity, free radical binding and metal ion titration to intracellular redox potential, are members of the metallothionein (MT) protein family. MTs are low molecular weight, single chain polypeptides with extraordinarily high content of the amino acid cysteine. It is the sulfhydryl groups of the cysteine residues, which enable MT proteins to bind metal ions, scavenge reactive species, display antioxidant activity, and respond to changes in intracellular redox potential (Babula et al., 2012).

In humans, MTs are grouped into four subgroups (MT1-4), with subgroup MT1 containing at least eight identified members, and subgroups MT2-4 containing one identified member each (Babula et al., 2012). With the active cysteine residues being conserved both in number and position, across all MT isoforms, differences between isoforms can only be found in structural amino acids separating the cysteines, as well as in regulatory gene regions controlling expression domains (Babula et al., 2012).

MT1 and MT2 are expressed in most soft tissues of the human body, with highest levels in liver and kidney, whereas MT3 is seen as brain-specific isoform, displaying highest expression levels in the nervous system, but also in heart, kidney and reproductive organs. MT4 isoform displays the most restricted expression pattern, and has so far only been found in squamous epithelial cells (Babula et al., 2012). Expression of MTs is induced by various stimuli like stress hormones (e.g. glucocorticoids), cytokines, radiation, heavy metal and chemical contamination, inflammation, and oxidative stress (Babula et al,, 2012). MT proteins can be detected both intra- and extracellularly, and are taken up and distributed throughout various body compartments via megalin/megalin-cubulin receptors (Leung et al., 2012; Atrian et al., 2013). Half-life time of MTs is dependent on both metallation status (i.e. how many binding sites are occupied by which metal ions) and redox status (i.e. how many free cysteine sulfur groups being oxidized) (Krezel et al., 2007). Degradation of MTs mainly takes place in lysosomes, and excretion of degradation products happens via bile and urinary tract (Bremner et al., 1987).

Based on their molecular functions of heavy metal binding, physiological metal ion titration, free radical inactivation, antioxidant and redox potential balancing, MTs have recently been linked to an array of medical conditions. In the context of cancer, restoring the pathologically silenced expression of MTs in e.g. hepatocellular carcinoma cells, colon cancer cells, papillary thyroid cancer cells or prostate cancer cells, has been demonstrated in vitro and in vivo, to block growth and metastatic potential (i.e. migration, adhesion, invasion) of the cancer cells, to induce their apoptosis, and to decrease tumor mass and volume (Mao et al., 2012; Yan et al., 2012; Fu et al., 2013; Arriaga et al., 2014). Molecular mechanisms involve balancing of free intracellular zinc levels, which has also been shown to make cancer cells more sensitive to chemotherapeutics (Arriaga et al., 2014).

Chronic oxidative stress in the central nervous system (CNS) is well accepted as underlying cause of age-related neuro-degenerative disorders like Parkinson's and Alzheimer's Disease (PD and AD, respectively) (Eibl et al., 2010). In particular, the neurotransmitter dopamine (DA) is highly reactive due to its chemical properties, and its free intra- and extracellular levels have to be tightly regulated (Eibl et al., 2010). Oxidized dopamine products (oxDA) are neurotoxic, and have been shown to covalently bind to a variety of cellular substrates, thereby obstructing their biological functions. Among the affected proteins is e.g. parkin, the gene of which is also highly associated to development of inherited familial PD. Arylation of parkin via oxDA products decreases its solubility and impairs its enzymatic activity (La Voie et al., 2005). Similar arylation, via oxDA species, of the dopamine transporter (which clears free dopamine from the synaptic cleft), has been demonstrated to inactivate the transporter. The dopamine left stranded in the synaptic space, is again auto-oxidized, resulting in further aggravation of the problem (Whitehead et al., 2001; Miyazaki et al., 2008). MTs have been shown to act as direct substrate for oxDA species, thereby helping to inactivate and clear out the neurotoxic oxDAs (Maret, 2006; Gauthier et al., 2008). Upon arylation of zinc-bound MT, zinc ions are released from MT, and zinc, in turn, is known to not only directly activate antioxidant Cu-Zn-SOD enzyme, but also activate transcription of SOD and catalase genes (Fattman et al., 2003). In this way, metallothioneins are further activating the cellular defense against oxDA mediated oxidative stress (Eibl et al., 2010). Additional pathological features of PD include mutation and oligomerization of α-Synuclein (α-Syn) protein. It has been shown in mouse models for PD, that artificial activation of MT can block oligomerization of α-Syn proteins, and can abolish the detrimental redox activity of α-Syn oligomers (Meloni et al., 2011). Mice with over-activated expression of MTs are resistant to development of PD in many different PD model situations (Sharma et al., 2013; Ebadi et al., 2005).

Pathological hallmarks of AD include copper-mediated aggregation of various amyloid-beta protein (β-AP) isoforms, extracellular accumulation of neurotoxic levels of free zinc within these β-AP plaques, as well as intra-neuronal accumulation of pro-oxidant and neurotoxic iron ions. All these detrimental, redox-potential-related features can be inhibited by metallothioneins. Administration of zinc-bound MT2, for example, has been shown to inhibit aggregation of β-AP isoforms via swapping zinc for copper ions, in mouse AD models (Chung et al., 2010). In a similar mechanism of swapping Zn²⁺ for Cu²⁺, Meloni et al. have proven, that Zn²⁺-MT3 can abolish ROS production and resulting neurotoxicity of β-AP plaques in AD mice (Meloni et al., 2008).

Chronic lifestyle- and malnutrition-induced oxidative stress has also been established as underlying cause for various metabolic disorders (Chimienti, 2013). Continuous overloading of intrinsic protection mechanisms against oxidative stress, leads to differentiation and development of new fat storage cells (adipocytes), as well as accumulation of lipids in these adipocytes. Increase of lipid content in white fat tissue, in turn, leads to further elevation of oxidative stress levels (Chimienti, 2013). MT proteins as antioxidants are well believed to be able to break this cycle of self-amplification, by buffering oxidative stress levels and boosting antioxidant protection mechanisms (Chimienti, 2013; Sato et al., 2010; Higashimoto et al., 2013). MT1 and MT2 have been demonstrated to protect mice against high-fat-diet-induced hyperlipidemia and obesity in vivo (Sato et al., 2010), as well as against high-fat-diet-induced oxidative and ER (endoplasmatic reticulum) stress inclusive resulting oxidative DNA damage (Higashimoto et al., 2013). Furthermore, metallothioneins protect against diabetic nephropathy and cardiomyopathy, both of which being diabetes-induced conditions based on elevated oxidative and ER stress (Tachibana et al., 2014; Guo et al., 2009; Ruttkay-Nedecky et al., 2013). There is also strong evidence, that zinc-bound MTs help stabilizing and normalizing aberrant insulin levels in diabetic patients, by fine-balancing free intracellular zinc levels in pancreatic islet β-cells (Chimienti, 2013). Well-balanced Zn²⁺levels in the pancreatic islets have now been demonstrated to be essential not only for biosynthesis and storage of insulin as well as maturation of insulin granules prior to release, but also for controlled glucose-stimulated insulin secretion (Slepchenko et al., 2015).

In view of the related art, further reference is made to the teaching of T. T. Ngu et al. in Biochemical and Biophysical Research Communications 401 (2010), pages 69-74*,* which refers to the arsenic transfer between metallothionein proteins at physiological pH and discloses isolated partially metallated apo-metallothionein proteins (apo-MT) and teaches a method for producing partially As-metallated species by protein-protein metal transfer, since As cannot metallate MT at pH 7. It further reveals the use of Cd₄-α-hMT, Cd₃-β-hMT and Cd₇-βα-hMT. Said Cd-bound proteins serve a precursor to be incubated at low pH with As-equivalents for obtaining partially metallated As³⁺-MT-species.

### 3. REFERENCE

Cadenas E., and Davies K.J.A. (2000). Mitochondrial free radical generation, oxidative stress, and aging. Free Radic. Biol. Med. 29: 222-230.
Swindell, W.R. (2011). Metallothionein and the Biology of Aging. Ageing Res. Rev. 10(1): 132-45.
Chimienti, F. (2013). Zinc, pancreatic islet function and diabetes: new insights into an old story. Nutrition Research Reviews 26: 1-11.
Furukawa, S., et al. (2004).Increased oxidative stress in obesity and its impact on metabolic syndrome. J. Clin. Invest. 114: 1752-61.
Özcan, U., et al. (2004).Endoplasmatic reticulum stress links obesity, insulin action, and type 2 diabetes. Science 306: 457-61.
Miyazaki, I., and Asanuma, M. (2008). Dopaminergic neuron-specific oxidative stress caused by dopamine itself.Acta Med. Okayama, 62 (3): 141-50.
Vasto, S., et al. (2008). Inflammation, genes and zinc in Alzheimer's disease. Brain Res. Rev., 58: 96-105.
Moreli, J.B., et al. (2014). DNA Damage and Its Cellular Response in Mother and Fetus Exposed to Hyperglycemic Environment. Biomed Res Int., Vol. 2014, Article ID 676758.
Jiang, L.J., et al. (1998). The glutathione redox couple modulates zinc transfer from metallothionein to zinc-depleted sorbitol dehydrogenase. Proc. Natl. Acad. Sci. USA 95: 3483-3488.
Aquilano, K., et al. (2014). Glutathione: new roles in redox signaling for an old antioxidant. Front Pharmacol. August, Vol. 5, Article 196.
Babula, B., et al. (2012). Mammalian metallothioneins: properties and functions. Metallomics 4: 739-50.
Leung, JY., et al. (2012). Metallothionein promotes regenerative axonal sprouting of dorsal root ganglion neurons after physical axotomy. Cell Mol Life Sci. 2012 March; 69(5):809-17.
Atrian, S., and Capdevila, M. (2013).Metallothionein-protein interactions. Biomol Concepts. 2013 April; 4(2):143-60.
Krezel, A., and Maret, W. (2007). Different redox states of metallothionein/ thionein in biological tissue.Biochem. J. 402: 551-8.
Bremner, I., et al. (1987). Metallothionein in blood, bile and urine.Experientia Suppl. 52: 507-17.
Mao, J., et al. (2012). Metallothionein MT1M is a tumor suppressor of hepatocellular carcinomas. Carcinogenesis 12: 2568-77.
Yan, D.W., et al. (2012). Downregulation of Metallothionein 1F, a putative oncosuppressor, by loss of heterozygosity in colon cancer tissue.Biochim. Biophys. Acta 2012, 1822, 918-926.
Fu, Y., et al. (2013). Metallothionein 1G functions as a tumor suppressor in thyroid cancer through modulating the PI3K/Akt signaling pathway. BMC Cancer 13: 462.
Arriaga, J.M., et al., (2014). Metallothionein 1G and zinc sensitize human colorectal cancer cells to chemotherapy. Mol Cancer Ther.2014 Mar 14.epub ahead of print.
Han, Y.C., et al. (2013). Metallothionein 1h tumour suppressor activity in prostate cancer is mediated by euchromatinmethyltransferase 1. J Pathol 2013; 230 (2),: 184-93.
Eib1, J.K., et al. (2010). Zinc-metallothionein: a potential mediator of antioxidant defence mechanisms in response to dopamine-induced stress. Can. J. Physiol. Pharmacol. 88: 305-312.
LaVoie, M.J., et al. (2005). Dopamine covalently modifies and functionally inactivates parkin. Nat. Med. 11(11): 1214-21.
Whitehead, RE., et al. (2001). Reaction of oxidized dopamine with endogenous cysteine residues in the human dopamine transporter. J. Neurochem. 76(4): 1242-1251.
Miyazaki, I., and Asanuma, M. (2008). Dopaminergic neuron-specific oxidative stress caused by dopamine itself.Acta Med. Okayama, 62(3): 141-150.
Maret, W. (2006). Zinc coordination environments in proteins as redox sensors and signal transducers. Antioxid. Redox Signal. 8(9-10): 1419-1441.
Gauthier, M.A., et al. (2008). Covalent arylation of metallothionein by oxidized dopamine products: a possible mechanism for zinc-mediated enhancement of dopaminergic neuron survival. Neurotox. Res. 14(4): 317- 328.
Fattman, C.L., et al. (2003). Extracellular superoxide dismutase in biology and medicine. Free Radic. Biol. Med. 35(3): 236-256.
Meloni, G., Vašák, M. (2011). Redox activity of α-synuclein-Cu is silenced by Zn7-metallothionein-3. Free RadicBiol Med. 50(11): 1471-9.
Sharma, S., et al. (2013).Biomarkers in Parkinson's disease (recent update).Neurochem Int. 63(3):201-29.
Ebadi, M., et al. (2005).Metallothionein-mediated neuroprotection in genetically engineered mouse models of Parkinson's disease. Brain Res Mol Brain Res. 134(1):67-75.
Chung, R.S., et al. (2010). The native copper- and zinc-binding protein metallothionein blocks copper-mediated Abeta aggregation and toxicity in rat cortical neurons. PloS One 5, e12030.
Meloni, G., et al. (2008). Metal swap between Zn7-metallothionein-3 and amyloid-beta-Cu protects against amyloid-beta toxicity. Nat. Chem. Biol. 4: 366-372.
Chimienti, F. (2013). Zinc, pancreatic islet cell function and diabetes: new insights into an old story. Nutr. Res. Rev. 26(1):1-11.
Sato, M., et al. (2010).Development of high-fat-diet-induced obesity in female metallothionein-null mice.FASEB J. 24; 2375-2384.
Higashimoto, M., et al. (2013).Preventive effects of metallothionein against DNA and lipid metabolic damages in dyslipidemic mice under repeated mild stress. J. Med. Invest. 60(3-4):240-8.
Tachibana, H., et al. (2014). Metallothionein deficiency exacerbates diabetic nephropathy in streptozotocin-induced diabetic mice. American Journal of Physiology-Renal Physiology, 306: F105-F115.
Guo, R., et al. (2009). Metallothionein Alleviates Oxidative Stress-Induced Endoplasmic Reticulum Stress and Myocardial Dysfunction. J. Mol. Cell Cardiol.47(2): 228-237.
Ruttkay-Nedecky, B., et al. (2013).The Role of Metallothionein in Oxidative Stress. Int. J. Mol. Sci. 14: 6044-6066.
Slepchenko, KG., et al. (2015). Autocrine effect of Zn(2+) on the glucose-stimulated insulin secretion. Endocrine. 2015 Sep; 50(1): 110-22.

### SUMMARY OF THE INVENTION

The present invention relates to a method for producing a hypo-metallated redox-active metallothionein protein. The hypo-metallated redox-active metallothionein protein has 20 cysteine sulfhydryl groups, thus forming 7 metal ion binding pockets. Two (2) to 16 of the 20 cysteine sulfhydryl groups are free and reduced, and 1 to 6 of the 7 binding pockets are occupied by metal ions. The method comprises the following steps:
providing a metallothionein protein;
de-metallating the metallothionein protein;
chemically reducing all the 20 cysteine sulfhydryl groups of the de-metallated metallothionein protein; and
partially metallating the reduced de-metallated metallothionein protein by providing 1, 2, 3, 4, 5, or 6 metal ions to the 7 binding pockets.

In some embodiments, the metallothionein protein has an amino acid formula of [Xₙ₁CXCXₙ₂CXCXₙ₃CXCXₙ₄CXCXₙ₅CXCXₙ₆CCXCCXₙ₇CXₙ₈CXₙ₉CXCXₙ₁₀CXCCXₙ₁₁] wherein X is any biogenic L-amino acid with the exception of cysteine, phenylalanine, tryptophan, and tyrosine, andn1 to n11 are integers of any value from 1 to 12.

In some embodiments, the method further comprises a step of fully pre-metallating the metallothionein protein before the step of de-metallating the metallothionein protein.

In some embodiments, the step of de-metallating the metallothionein protein is performed by acidifying the metallothionein protein to a pH value below pH4.

In some embodiments, the method further comprises a step of purifying the de-metallated metallothionein protein before the step of chemically reducing the de-metallated metallothionein protein. In some embodiments, the step of purifying the de-metallated metallothionein protein is performed by one of dialysis, cation-exchange chromatography, reverse-phase chromatography, high-pressuxe-liquid chromatography, electrophoretic methods, gel filtration, and magnetic force.

In some embodiments, the step of chemically reducing the de-metallated metallothionein protein is performed by adding a reducing agent. In some embodiments, the reducing agent is one of glutathione, reduced nicotinamid adenine dinucleotide (NADH), nicotinic acid, and dithiothreitol (DTT).

In some embodiments, the metal ions are selected from the group of zinc, copper, iron, and selenium.

The hypo-metallated redox-active metallothionein protein produced by the method described above can be used for a pharmaceutical composition, which comprises at least one such hypo-metallated redox-active metallothionein protein. The at least one hypo-metallated redox-active metallothionein protein has 20 cysteine sulfhydryl groups and 7 metal ion binding pockets, 2 to 16 of the 20 cysteine sulfhydryl groups are free and reduced, and 1 to 6 of the7 binding pockets are occupied by metal ions.

It is noted that the hypo-metallated redox-active metallothionein protein produced by the method described above may be used for the manufacture of a medicament for treatment of a condition originating from elevated intracellular oxidative stress, dis-balanced intracellular redox-potential, or redox-potential-dependent imbalance of metal ions.
The condition originating from elevated intracellular oxidative stress, dis-balanced intracellular redox-potential, or redox-potential-dependent imbalance of metal ions can be cancer, neurodegenerative diseases, or metabolic disorders. The cancer is at least one of hepatocellular carcinoma, colon cancer, prostate cancer and/or papillary thyroid cancer. The neurodegenerative diseases may be Parkinson's disease, Alzheimer's disease, Wilson's Disease, Amyotrophic Lateral Sclerosis (ALS), and/or Huntington's Disease. The metabolic disorders may be diabetes mellitus type I, diabetes mellitus type II, hyperlipidemia, obesity, and/or fatty liver syndrome.

These and other aspects of the present invention will become apparent from the following description of the preferred embodiment taken in conjunction with the following drawings, although variations and modifications therein may be effected without departing from the spirit and scope of the novel concepts of the disclosure.

### BRIEF DESCRIPTION OF THE DRAWINGS

The accompanying drawings illustrate one or more embodiments of the invention and together with the written description, serve to explain the principles of the invention. Wherever possible, the same reference numbers are used throughout the drawings to refer to the same or like elements of an embodiment, and wherein:
FIG. 1 shows molar zinc contents of metal-free metallothionein (MT) apo-proteins ("apo-MT1a" and "apo-MT2a"), fully metallated MT proteins ("7Zn-MT1a" and "7Zn-MT2a"), and hypo-metallated redox-active MT protein ("hypo-MT1a" and "hypo-MT2a").
FIG. 2 shows redox status of oxidized metallothionein (MT) apo-proteins ("OxApo-MT1a" and "OxApo-MT2a") and hypo-metallated redox-active MT protein ("hypo-MT1a" and "hypo-MT2a").
FIG. 3A shows redox-dependent zinc release of metal-free MT apo-proteins ("apo-MT1a" and "apo-MT2a") and hypo-metallated redox-active MT protein ("hypo-MT1a" and "hypo-MT2a"), under oxidative conditions.
FIG. 3B shows redox-dependent zinc binding of hypo-metallated redox-active MT protein under redox-neutral conditions ("neutral-MT1a" and "neutral-MT2a") and hypo-metallated redox-active MT protein under reductive conditions ("hypo-MT1a" and "hypo-MT2a").
FIG. 4 shows capability of stabilizing metabolic integrity (as measure of redox-potential balancing), of control MT proteins ("controlMT1a" and "controlMT2a") and hypo-metallated redox-active MT protein ("hypo-MT1a" and "hypo-MT2a") under oxidative stress conditions.

### DETAILED DESCRIPTION OF THE PREFERRED EMBODIMENT

The invention provides a biotechnological method for producing hypo-metallated redox-active metallothionein (MT) proteins, which according to the invention will have defined numbers of MT cysteinyl binding sites occupied with physiologically relevant metal ions (preferably zinc Zn²⁺, as predominant physiological ligand), as well as having additional defined numbers of MT cysteinyl binding sites unoccupied and being in a chemically reduced (i.e. non-oxidized) state.

The hypo-metallated redox-active MT proteins obtained by the method described herein share the amino acid composition and structural motifs conserved in all known naturally occurring mammalian metallothioneins, with the amino acid cysteine, as being essential for the biological activity of said proteins, being conserved in both number and intra-molecular position. Such proteins preferably are 61 to 68 amino acids long and contain 20 cysteine residues at conserved positions, thus forming seven redox-active binding pockets for divalent metal ions. Structural amino acids comprising the rest of said proteins may include all other naturally occurring biogenic amino acids with exception of cysteine and aromatic amino acids phenylalanine, tryptophan and tyrosine.

The hypo-metallated redox-active MT proteins, after being produced according to the invention, bind no less than one, and no more than six physiologically relevant metal ions, such as zinc, iron, copper or selenium. Hence, the number of metal ions per protein molecule can range from 1 to 6.

The hypo-metallated redox-active MT proteins, after being produced, purified and partially metallated with preferred metal ions, according to the method of the present invention, contain un-occupied cysteine binding pockets comprised of cysteine residues with non-oxidized (i.e. chemically reduced) sulfhydryl groups. When the protein contains 1, 2, 3, 4, 5, or 6 un-occupied cysteine binding pockets, each protein has 2, 5, 9, 12, 13, or 16 free and reduced cysteine sulfhydryl groups, respectively. Hence, the number of free and reduced cysteine sulfhydryl groups per protein molecule can range from 2 to 16.

As per current scientific data, physiologically significant forms of metallothionein proteins are the fully metallated isoform, as well as the oxidized and reduced metal-free apo-proteins (Krezel et al., 2007, see above). Intermediate forms, either partially oxidized or partially metallated have never been detected or isolated in vivo.

From functional point of view, fully oxidized metallothionein apo-proteins would not be able to scavenge free radicals, display anti-oxidant activity, or bind or release metal ions. A fully reduced metallothionein apo-protein would be able to act as anti-oxidant and could bind metal ions, but logically could not release metal ions in response to redox changes. In addition, a fully metallated metallothionein protein could release metal ions in response to redox changes and act as anti-oxidant, but it would not be able to bind additional metal ions. Consequently, only a hypo-metallated MT protein with its free cysteine residues in reduced form would be situated to act in all three ways, as anti-oxidant, as metal ion binding and metal ion releasing redox-responsive molecule.

Also, from the point of view of "intra- and extracellular fine-balancing of free zinc levels" (and all the downstream effects thereof), only a partially metallated MT protein can act as both "sink" and "source" of zinc ions. In response to changes in redox potential, fully metallated MT proteins could not lower free zinc levels, whereas metal-free metallothionein apo-proteins could not release zinc ions.
Taken together, the present invention functionally activates naturally occurring or artificially designed MT proteins to be able to act in these specific ways, by generating hypo-metallated MT proteins in specific metallation and redox states. In contrast, the naturally occurring forms of metallothionein proteins, in their naturally occurring metallation and redox states, are not able to function in the same way.

The hypo-metallated MT proteins obtained by the method of the present invention may be further used for pharmaceutical compositions containing these hypo-metallated redox-active MT proteins. The hypo-metallated redox-active MT proteins retain their full, unimpaired metal ion load, their redox-potential responsiveness and their antioxidant capacity based on their reduced cysteine sulfhydryl groups.

The hypo-metallated redox-active MT proteins obtained by the method of the present invention may be used for preventive and curative treatment of any conditions originating from elevated intracellular oxidative stress, dis-balanced intracellular redox-potential, or redox-potential-dependent imbalance of metal ions. Accordingly, the pharmaceutical compositions containing the hypo-metallated redox-active MT proteins may be used in the treatment of conditions, where normalizing and stabilizing intracellular redox-potential, oxidative stress levels, and free levels of redox-potential-linked metal ions, are beneficial and supportive for the treatment. Such conditions include but are not limited to: various forms of cancer, such as hepatocellular carcinoma, colon cancer, prostate cancer and papillary thyroid cancer; various neurodegenerative diseases, such as Parkinson's disease, Alzheimer's disease, Wilson's Disease, Amyotrophic Lateral Sclerosis (ALS), Huntington's Disease; various metabolic disorders, such as Diabetes mellitus types, Hyperlipidemia, Obesity, Fatty Liver Syndrome.

### Production Of Metallothionein Apo-Proteins

The metallothionein apo-proteins, in their native form with undefined metallation status and undefined redox-status of their cysteine sulfur residues, may be produced by any conventional method of recombinant DNA technology, protein synthesis, enzymatic cleavage of pre-pro-proteins, or isolation of naturally occurring variants of mammalian metallothionein proteins.

Thus, the metallothionein apo-proteins can be produced by established recombinant DNA technology (Sambrook et al., Molecular Cloning: A Laboratory Manual, 2nd Edition, Cold Spring Harbor, 1989, and references therein). The DNA sequence encoding the respective full-length metallothionein protein isoform may either be synthesized by standard nucleic acid synthesis methods, or may be obtained by established laboratory methods of directly isolating it from appropriate genomic or cDNA libraries, or indirectly by copying and amplifying it from said libraries by standard polymerase chain reaction (PCR) method.

The DNA sequence, after appropriate preparation and purification, may then be introduced into a recombinant expression vector of choice, using, again, established standard methods. The choice of an appropriate vector may depend on the choice of protein expression system to be used. The expression systems, as established and extensively described in scientific literature well known to the person skilled in the art, include prokaryotic bacterial cells, eukaryotic yeast and fungal cells, as well as cell cultures derived from higher animals such as insects, arthropods or mammals. Introduction of the protein expression vector, equipped with the coding sequence for the chosen MT protein variant, into appropriate host cells, may be facilitated by established protocols.

The choice of protein expression system and compatible recombinant expression vector into which the described coding DNA for any chosen metallothionein variant is introduced, may also determine features like
choosing extra-chromosomal plasmid vectors versus vectors that integrate into host cell genomes;
choosing and including appropriate promoter sequences for transcriptional activation as well as transcriptional terminator sequences;
choosing and including origin-of-replication sequences and selection marker sequences; and
the choice and inclusion of sequences for fusion proteins and protease cleavage sites appropriate for the chosen protein expression and purification methods.

All these and additional features concerning established methods of protein expression, are well known to the person skilled in the art, and may be decided upon relating to established standard biotechnology methods.

Thus, in accordance with the above, in one example the yeast-based protein expression system *Kluyveromyceslactis* (*K*. *lactis*) (New EnglandBiolabs Inc., MA, USA) can be used.

The coding regions of mammalian (e.g. bovine or human) isoforms of metallothioneins may be PCR-amplified from appropriate cDNA libraries, using standard synthesized PCR primers specific for the respective gene of interest and including restriction sites such as *Xho*I and *Not*I as well as the Kex protease cleavage site. After PCR amplification of the desired DNA fragments according to the manufacturers protocols, the DNA fragments may be cloned into the manufacturers pKLAC2 plasmid vector using *Xho*I and *Not*I restriction endonucleases and DNA ligase I. After appropriate purification as well as linearization of said vector using restriction endonuclease *Sac*II and the compatible restriction site intrinsic to the pKLAC2 vector, competent *K*. *lactis* cells provided by the manufacturer may be transformed with said linearized expression cassette. Using positive and negative selection markers intrinsic to the pKLAC2 vector, positive clones of *K*. *lactis*, containing individual or multiple copies of the desired metallothionein expression cassette correctly integrated into the host genome, may be selected using the manufacturers protocols. Thus, in this example, said stably transformed *K*. *lactis* cells may then be grown in appropriate culture medium, to stably express the chosen metallothionein variant as a fusion protein fused to the intrinsic *K*. *lactisa*-mating factor domain (α-MF) under transcriptional control of the intrinsic *K*. *lactis* LAC4 promoter, and to secrete, after intrinsic Kex protease cleavage of α-MF, the native and post-translationally modified form of the desired metallothionein protein, with unspecified metallation status and unspecified redox-status of cysteine sulfur residues.

### Purification, Reduction and Hypo-Metallation Of Metallothionein Proteins

It is one key objective of present invention to provide metallothionein proteins with defined numbers of metal ions coordinately bound to their cysteine residues, as well as with defined numbers of free cysteine sulfur residues being present in chemically reduced sulfhydryl (-SH) form. Therefore, preferred embodiments of the invention utilize suitable methods for producing such hypo-metallated redox-active metallothionein proteins, in which the above-described metallothionein apo-proteins maybe completely de-metallated, chemically reduced at their cysteine sulfhydryl groups, and then partially metallated with defined amounts of the preferred physiologically relevant metal ions like zinc, copper, iron or others.

As per present invention, native forms of chosen metallothionein apo-proteins, with un-specified metallation and redox-status, produced by any of the above-described methods of recombinant DNA technology, protein synthesis, enzymatic cleavage of pre-pro-proteins, isolation of variants of the metallothionein proteins, or alternative methods, may first be isolated and purified from the respective culture medium, buffer solution or biological substrate.

According to the invention, this initial isolation and purification may be achieved by any standard protein biochemistry method well known to the person skilled in the art. Established methods like dialysis against appropriate aqueous buffers, cation-exchange chromatography, reverse-phase chromatography, high-pressure-liquid chromatography, electrophoretic methods, gel filtration etc., may be chosen with respect to the previously chosen production method of the MT apo-proteins (as described above) and the given condition of said MT apo-proteins. Isolation of the respective MT proteins may also be achieved by using magnetic force, in which case the MT proteins may first be fully pre-metallated with e.g. zinc ions. Such pre-metallation, with the purpose of enabling initial isolation and purification, is to be distinguished from final binding of defined amounts of chosen physiological metal ions, in order to produce the hypo-metallated end product of present invention.

One example of this invention uses the above-mentioned recombinant DNA method of MT protein expression in the yeast *Klyuveromyces lactis*. In this example, the chosen MT isoform, as apo-protein, will be secreted into the respective culture medium, and will stay in solution, with un-defined metallation and redox status. After separation of yeast cells from culture medium, by standard methods like centrifugation or filtration etc., the protein concentration of said MT solution may be determined using standard well-established methods like Bradford Protein Assay etc. This, in turn, will allow for calculation of the required maximal amount of metal ions that can be bound by the present MT proteins, assuming that all MT binding sites are un-occupied. Since each mole of MT protein can bind up to seven moles of divalent metal ions, the appropriate amount of zinc salt (e.g. as zinc sulfate or zinc chloride, etc.) may be added to the MT solution, in order to allow for full pre-metallation. From this solution, the fully reconstituted Zn₇-MT protein may be recovered using magnetic force.

Other examples may make use of other standard, well established isolation and purification methods, and may lead to MT protein isolates with other or un-defined pre-metallation status.

Any of the MT protein isolates, independent of the metallation status of MT proteins contained therein, may then be subjected to acidification, to any pH value below pH4, in order to allow for complete dissociation of all bound metal ions. In preferred embodiments of this invention, any naturally occurring monovalent acid with a pKₐ value between 1.27 and 4.76 (formic acid, acetic acid, oxalic acid, ascorbic acid, or others) may be chosen, and acidification to below pH4 might either be achieved by step-wise addition of the respective acid, followed by standard measurement of pH value of the solution, until a pH value below pH4 is reached, or by calculating the necessary amount of hydrogen ions (hydronium ions in aqueous solutions, respectively) based on the concentration of MT protein in solution (see above). For example, in such a calculation, 20 moles of hydrogen ions are required per mole of MT protein, to saturate all previously metal-bound cysteine sulfur residues with hydrogen ions, assuming that the respective MT proteins have been fully pre-metallated.

As per one of the key objectives of present invention, which is the partial metallation of the MT proteins with defined numbers of specific metal ions, the above-described MT protein isolates, after complete de-metallation, may now be purified from any metal ion, inclusive the metal ions that might have been used for pre-metallation (see above). This purification, dependent on the embodiment of present invention, may be achieved by any standard protein biochemical method, for example by dialysis against appropriate metal-free buffer solution. In other embodiments, electrophoretic methods, or magnetic force may be used to remove said metal ions. In any chosen method of metal ion removal, the pH value of the given MT protein solution is to be kept at below pH4, in order to ensure the metal-free status of said MT proteins, and to enable complete removal of any said metal ions.

According to present invention, the respective MT protein isoforms may now, after above-described isolation, purification, and de-metallation, be subjected to chemically reducing conditions in order to ensure chemical reduction of any potentially oxidized cysteine sulfur residues. In preferred embodiments of present invention, this may be achieved by adding necessary amounts of appropriate reducing agents, including but not limited to glutathione, reduced nicotinamid adenine dinucleotide (NADH), niacin (nicotinic acid), dithiothreitol (DTT)or other biochemical electron donors, to the described MT protein solution. The amounts of reducing agent necessary to chemically reduce all present cysteine sulfur residues, may again be calculated from the above-mentioned protein concentration, and based on the assumption, that all cysteine sulfur residues might be oxidized. For example, one mole of MT protein in the solution may thus require 20 moles of monovalent reducing agent for complete reduction of all MT cysteine sulfur residues to sulfhydryl (-SH) form.

In accordance with present invention, above-described MT protein isoforms, which are now completely de-metallated and completely reduced at their cysteine sulfur residues, and are still in acidic solution under reducing conditions, may now be partially metallated with defined amounts of the chosen metal ion, for example zinc ions. In one preferred embodiment of the invention, the given MT isoform may be reconstituted to partially metallated Zn₄-MT form, with four divalent zinc ions bound per one MT molecule. The necessary amount of zinc ions may be calculated based on MT isoform concentration in solution, and four molar equivalents of zinc ions may be added to said solution, preferably as zinc sulfate or zinc chloride. In order to allow reconstitution of the partially metallated Zn₄-MT protein isoform, the pH value of said solution may be elevated from previously acidic to neutral, with pH values between pH 6.5 and pH 7.5 being considered as neutral. This may be achieved by adding necessary amounts of neutralizing agents like ammonium hydroxide, ammonium acetate, or others, to said solution, and consecutive measurement of the solution's pH value, using standard pH measurement methods, until said neutral pH value is reached. Thus, in this embodiment of the invention, a partially Zn-metallated MT protein isoform, bound to four zinc ions per molecule and with its free cysteine sulfhydryl groups being chemically reduced and therefor readily redox-active, may be produced.

In other embodiments of the current invention, different metallation states, with only one to three zinc ions bound per molecule and respectively more cysteine sulfhydryl residues being reduced and redox-active, or with even five or six zinc ions bound per molecule and respectively less cysteine sulfhydryl groups being reduced and redox-active, or hypo-metallation with physiological metal ions other than zinc (such as copper, iron, selenium, or others), may be produced in analogous manner. Thus, in an additional embodiment of the invention, partial metallation with copper ions may be achieved by replacing zinc salts with copper salts in above-described method, whereas in yet other embodiments different metallation states with any chosen metal ion may be achieved by adapting the necessary amounts of the respective metal salts.

According to present invention, the chosen MT protein isoform, which is now partially metallated with defined numbers of specific metal ions, and is now redox-active with defined numbers of cysteine sulfur residues present as reduced sulfhydryl groups, may now be isolated and purified, in order to allow for subsequent manufacturing into below-described compounds. In preferred embodiments of the invention, the final purification may be done by any established protein biochemical method that allows for un-impaired metallation and redox status of said MT protein isoforms. Accordingly, during this final purification step, pH values below pH4, as well as chemically oxidizing conditions are to be avoided.

### Metallothionein Proteins

The metallothionein proteins described herein share amino acid composition and structural motifs conserved in all known naturally occurring mammalian metallothioneins, with the amino acid L-cysteine, as essential for the biological activity of said proteins, being conserved in both number and intra-molecular position. Such proteins preferably are 61 to 68 amino acids long, and contain exactly 20 cysteine residues at conserved positions, thus forming exactly seven redox-active binding pockets, each of which allows for coordinated binding of one divalent metal ion. The amino acids comprising the rest of said proteins may include all other naturally occurring biogenic L-amino acids with exception of cysteine and the aromatic amino acids phenylalanine, tryptophan and tyrosine.

In one embodiment, the method of the present invention relates to the making of metallothionein proteins with the amino acid formula: [Xₙ₁CXCXₙ₂CXCXₙ₃CXCXₙ₄CXCXₙ₅CXₙ₆CCXCCXₙ₇CXₙ₈CXₙ₉CXCXₙ₁₀CXCCXₙ₁₁], wherein "X" may be any biogenic L-amino acid with the exception of cysteine ("C") and the aromatic amino acids phenylalanine, tryptophan and tyrosine, and wherein "n1" to "n11" may be integers of any value from 1 to 12. Thus, in this aspect of the invention and depending on the embodiment, the proteins may be 38 to 159 amino acids long, and contain no more and no less than 20 L-cysteines at conserved positions according to above formula, and contain no aromatic amino acids. The amino acid sequences of metallothionein proteins may be naturally occurring or artificially synthesized. In some examples, the metallothionein proteins are human metallothionein 1a protein (MT1a) having the amino acid sequence of SEQ ID NO: 2 and/or bovine metallothionein 2a protein (MT2a) having the amino acid sequence of SEQ ID NO: 4.

In yet another example, the present invention relates to proteins comprised of a single chain of amino acids, and relates to monomers of the proteins. However, in relation to an additional aspect of the invention, the proteins, after being hypo-metallated and redox-activated according to present invention, might also dimerize or multimerize as homo- or heteromers upon being manufactured into biologically active compounds and pharmaceutical compositions (as described below).

The invention also relates to naturally occurring or artificially introduced modifications of the free carboxy-terminus and amino-terminus of said proteins, as well as to naturally occurring or artificially introduced modifications of intra-molecular amino acid side chains, such as e.g. glycosylation, acetylation, myristoylation or others.

In the present context, terms like "protein isoform," "amino acid composition," "structural motifs," "conserved," "binding pockets," and other terms concerning amino acid and protein structure and function, are used in accordance with well-accepted scientific standards well known to the person skilled in the art.

### Compound

A compound may contain any biologically active amount of any combination of possible MT protein isoforms, of any metallation and redox status as described above, or pure compositions thereof.

This means that compounds comprising the MT protein isoforms produced according to this invention may be formulated as protein isoform specific compounds, containing only one given MT protein isoform, or as mixes of said MT protein isoforms, containing any possible combination of MT protein isoforms produced according to the invention.

It also means, that compounds comprising the MT protein isoforms produced according to this invention, may contain MT protein isoforms of only one specific metallation status, with only one species of metal ion bound at only one specific molar equivalent, or may contain mixes of said MT proteins, with more than one species of metal ion bound at the same or more than one specific molar equivalent. It is therefore also invention possible example, that compounds may contain said MT proteins of only one specific redox-state, or may contain mixes of said MT proteins with various specific redox-states.

Furthermore, a compound comprising the MT protein isoforms produced according to the invention may contain said hypo-metallated redox-active MT protein isoforms as monomers, homo- or heterodimers, or homo- or hetero-multimers.

Therefore in some examples, one specific compound may contain only one specific isoform of MT protein, with only one species of metal ion bound to said protein, at one specific molar equivalent, allowing for only one specific redox-status of said protein. However, as described above, specific combinations of MT protein isoforms of various metallation and redox-states are possible as well.

### Pharmaceutical Composition

Pharmaceutical compositions may be provided comprising one or more of the above-defined compounds, wherein the compounds contain the above-described hypo-metallated redox-active MT proteins with unimpaired metal load, redox-status and biological activity, and are capable of balancing intracellular redox potential, of scavenging free radical species causing oxidative stress, and of balancing free metal ion levels in response to redox potential. Thus, the MT protein isoforms produced according to the invention can be utilized to provide pharmaceutical compositions based on the described hypo-metallated redox-active MT proteins, wherein the compositions are, upon administration to a subject, beneficial for treatment of the subject's conditions originating from and manifesting in elevated oxidative stress, intracellular redox-potential imbalance, and redox-dependent imbalance of free metal ion levels.

A pharmaceutical composition, which in present context may synonymously be called "medicament," comprises an effective amount of one or more of the above-described compounds, in their pure version or in combination with pharmaceutically acceptable additives, such as vehicle, diluent, or carrier. In one example the medicament may be formulated for oral administration. However, in additional examples, the medicament may also be formulated for intravenous, percutaneous, or other routes of administration. Formulating the medicament for any given route of administration may also predetermine the choice of pharmaceutical additives to be added to the medicament, as well as the amount and concentration of pharmaceutically active ingredient. Formulation strategies for protein-based pharmaceutical compositions are established, extensively described in scientific literature, and well known to the person skilled in the art (Banga A. K., Therapeutic Peptides and Protein Formulation: Processing and Delivery Systems, Technomic Publishing AG, Basel 1995, and references therein).

Thus, pharmaceutical compositions comprising the MT protein isoforms produced according to the method of the present invention may be formulated for oral administration, with addition of standard excipients like mannitol, lactose or starch, cellulose, and other compatible additives, which may not impair the metallation and redox status of the active ingredients. Such oral formulations may take the form of pills, tablets, sustained release formulations, capsules, powders, solutions, suspensions or emulsions, and the like, and may generally contain 10-95% of the described active ingredients, typically 25-75%. Auxiliary substances, which might help improve absorption, transportation and/or effectiveness of said pharmaceutical composition, may be added at minor concentrations.

In another example the compositions may be formulated for injection or percutaneous application, in which case appropriate suitable excipients like water, saline, dextrose or glycerol may be added. Again, formulation of the pharmaceutical compositions for these or any other chosen route of administration may not in any way impair the metallation and redox status of the active ingredients. Guidelines concerning such formulation strategies as well as all kinds of possible additives, are published and well known to the person skilled in the art.

The described formulations containing the described active ingredients may be administered in a manner compatible with the dosage formulation, and in a therapeutically effective amount. The therapeutically effective amount of administered active ingredient may depend on the subject to be treated, the age and body weight of said subject, the condition to be treated and the stage of said condition, as well as on the route of administration. The route of administration, as well as duration of treatment and dosage, may again depend on age and body weight of the subject to be treated, as well as on condition to be treated, and stage of the condition. In one example the the described active ingredients may be used in a preventive way and continuous low-dosage administration regimens may be formulated according to the subject's situation.

### Biological Activity

Hypo-metallated and redox-active MT proteins obtained by the method of the present invention, a compound comprising the proteins, as well as pharmaceutical compositions comprising the compounds and therefore the proteins, possess biological activity as described above. The invention preferably relates to a method for obtaining hypo-metallated and redox-active MT proteins having biological activity such as the capability to bind and release physiological metal ions in response to changes of intracellular redox potential, to bind and scavenge redox-active heavy metal ions, to scavenge and inactivate free radicals such as ROS, RNS and others, and thereby exert antioxidant functions as well as balance the intracellular redox potential at physiological levels.

More specifically, said biological activity relates to the capability to bind and release zinc ions in response to changes of the intracellular redox potential, such as e.g. the changes of redox potential induced by elevated oxidative stress. Such capability of binding and/or releasing of zinc ions from the described MT proteins, in response to the intracellular redox potential, may be interpreted as capability of balancing the intra- and extracellular levels of free zinc.

In this context, oxidative stress, and thereby the changes in intracellular redox potential, may be induced by free radicals such as reactive oxygen species, reactive nitrogen species, superoxide anion radicals, or any other free radical containing one or more unpaired electrons.

Furthermore, biological activity also relates to the capability of antioxidant activity, in which the oxidative stress-inducing free radicals are bound, and thereby inactivated (synonymously also described as "scavenged"), via the free cysteine sulfhydryl residues of the described MT proteins. Such scavenging of free radicals will consequently also balance the intracellular redox potential, and therefore the capability of balancing the intracellular redox potential is seen as another aspect of biological activity.

Additionally, biological activity herein also relates to the capability of coordinative binding of heavy metals such as cadmium, lead or others, and thereby scavenging and biologically inactivating said heavy metal ions.

The hypo-metallated and redox-active MT proteins obtained by the method of the present invention may also possess another biological activity such as the capability of binding and/or releasing of physiological metal ions other than the above-described zinc ions, such as e.g. copper or iron ions.

The hypo-metallated and redox-active MT proteins obtained by the method of the present invention may also possess biological activities such as the capability of regenerating other intracellular antioxidants such as glutathione, NADH or others, via redox-coupling, in which the oxidized forms of the antioxidants are chemically reduced by the redox-active cysteine sulfhydryl groups of the described MT proteins. This might again be interpreted as the capability of balancing intracellular redox potential, as already described above.

The hypo-metallated and redox-active MT proteins obtained by the method of the present invention may also possess as biological activity the capability to directly bind and inactivate oxidized dopamine products, or any other product oxidized by above-described oxidative stress-inducing free radicals. This includes the regeneration of the non-oxidized version of said cellular component, via the redox-active cysteine sulfhydryl groups of described MT proteins.

The term "redox activity" refers to the capability of participating in biochemical reduction-oxidation reactions (commonly termed "redox reactions"), in which reducing agents are chemically oxidized by donating electrons, and oxidizing agents are chemically reduced by accepting electrons. The described MT proteins are redox-active in that they are capable of participating in such redox reactions as both reducing agents, by donating electrons from their free and reduced cysteine sulfhydryl groups, as well as redox-dependently binding or releasing zinc ions.

It is one key objective of present invention, to combine all the above-described biological activities of metal ion binding and balancing, antioxidant and scavenging activity, as well as redox-activity and capability of redox-potential-balancing, in one and the same protein, the described hypo-metallated redox-active MT proteins obtained by the method of the present invention.

In additional examples, the hypo-metallated redox-active MT proteins obtained by the method of the present invention further may have biological activities, which might be seen as mechanistically "downstream" of, or "secondary" to, the above-described activities. Such indirect biological activities include e.g. the capability of inhibition and/or suppression of inflammation, the capability of inhibiting and/or suppressing autoimmune reactions, or the capability of regulating gene activity. Such biological activities might be called "secondary" or indirect, for they are, mechanistically speaking, based on the above-described "primary" functions of the MT proteins, such as balancing free metal ion levels, antioxidant and scavenging activity, redox-activity or capability of redox-potential-balancing.

The hypo-metallated redox-active MT proteins obtained by the method of the present invention further may have biological activities, such as the capability of inducing cell cycle arrest and apoptosis in malignant cells like various cancer cells, or the capability of inhibiting metastatic properties, such as the ability of migration and invasion, of such cancer cells. Such biological activities also include the capability of stimulating and promoting nerve cell regeneration, as extensively described in e.g. U.S. patent 8,618,060 and references cited therein. The molecular mechanisms are scientifically proven to be directly or indirectly attributable to the above-described biological activities of MT proteins.

The above-described biological activities of the MT proteins may be exerted in any physiological compartment of the human body, where said activities are beneficial for treating or preventing the described conditions. Any formulation of the described MT proteins into described pharmaceutical compositions, as well as any route of administration of the proteins to the body compartments, which allows for full conservation of the above-described biological activities, can be applied.

### Treatment

The term "treatment" used herein relates to both preventive and curative treatment of subjects with MT proteins obtained by the method of the present invention, compounds or pharmaceutical compositions comprising the same, whenever above-described direct or indirect biological activities of the hypo-metallated redox-active MT proteins, such as balancing intra- and extracellular free metal ion levels, antioxidant activity, scavenging activity, redox-activity, capability of redox-potential-balancing, and others, are beneficial to treatment.

Such treatment relates to any applicable formulation of the described medicament, to any applicable route of administration of the medicament, as well as to any applicable treatment regimen with the medicament, depending on the subject and the condition to be treated. The term "applicable" refers to unimpaired biological functions, as defined above, of the proteins obtained by the method of the present invention, compounds and pharmaceutical compositions comprising the same.
Well known to the person skilled in the art, an extensive body of experimental and clinical data proves a direct link between elevated oxidative stress (as well as imbalanced intracellular redox-potential) and an array of clinical conditions such as various cancers, metabolic diseases or neurodegenerative diseases, with oxidative stress being both underlying cause and/or immediate primary effect or symptom of said conditions. Therefore the proteins obtained by the method of the present invention, compounds and pharmaceutical compositions comprising the same may be used for curative and preventive treatment of any such clinical condition that can be linked to elevated oxidative stress and imbalanced intracellular redox-potential.

Specifically the medicament may be used for treatment of conditions such as Parkinson's disease, Alzheimer's disease, Huntington's disease, amyloid lateral sclerosis (ALS), Wilson's disease, dementia, and other related neurodegenerative diseases.

The medicament may be also used for treatment of conditions such as hyperlipidemia, obesity, diabetes mellitus type 1 and 2, fatty liver syndrome, and other related metabolic disorders.

Furthermore the medicament may be used for treatment of conditions such as hepatocellular carcinoma, papillary thyroid cancer, colon cancer, and related cancer conditions.

In the context of any of the above-mentioned conditions, the medicament may be used for curative or preventive treatment of the respective condition, inclusive curative or preventive treatment of the underlying causes of said conditions, as well as for curative or preventive treatment with respect to effects, manifestations and symptoms of said conditions, by administering a protein, compound or pharmaceutical composition as defined herein.

As used in the description herein and throughout the claims that follow, the meaning of "a", "an", and "the" includes plural reference unless the context clearly dictates otherwise. Moreover, titles or subtitles may be used in the specification for the convenience of a reader, which shall have no influence on the scope of the present invention.

The meaning of the technical and scientific terms as described herein can be clearly understood by a person of ordinary skill in the art.

The present invention is more particularly described in the following examples that are intended as illustrative only since numerous modifications and variations therein will be apparent to those skilled in the art. Various embodiments of the invention are now described in detail. Referring to the drawings, like numbers indicate like components throughout the views.

### EXAMPLES

### 1. Production of hypo-metallated redox-active MT proteins.

MT proteins were produced by using the "*K*. *lactis* Protein Expression System" (New England Biolabs Inc., MA, USA). Respective cDNAs for human MTla (SEQ ID NO: 1) and bovine MT2a (SEQ ID NO: 3) were cloned into pKLAC2, and *K. lactis* cells were transfected, according to the manufacturers protocols. From the supernatants, the respective MT protein isoforms were pre-purified by centrifugation and over-night dialysis against standard Tris-HCl buffer at pH7. De-metallation was achieved by acidification to pH3.5 with formic acid, followed by dialysis against formic acid. After chemical reduction of all cysteine sulfur residues with 25 mM DTT, aliquots of metal-free reduced apo-proteins ("apo-MT1a" and "apo-MT2a") were removed for subsequent use as control samples. As representative examples for one preferred embodiment of the invention, the main protein fractions were then partially metallated with 4 molar equivalents of zinc ions ("hypo-MT1a" and "hypo-MT2a"), by adding respective amounts of zinc chloride, based on Bradford protein quantification. After over-night dialysis against Tris-HCl pH6.5, samples were subjected to analysis. Additional control samples of fully metallated proteins ("7Zn-MT1a" and "7Zn-MT2a") were generated by addition of 7 (rather than 4) molar equivalents of zinc chloride at the above reconstitution step.

### 2. Hypo-MTs bind defined amounts of metal ions.

Metal ion content of all samples and controls was determined as published (Ma, R.L., et al., 2004, Speciation of protein-bound trace elements by gel electrophoresis and atomic spectrometry. Electrophoresis 25(15): 2469-77). In brief, equimolar aliquots of samples and controls were dried, digested in nitric acid, and after dilution in deionized water, analyzed by inductively coupled plasma optical emission spectrometry (ICP-OES). Results from four independent experiments are displayed as molar equivalents of Zn²⁺ per mole of protein, and standard deviations are included in the graphs.

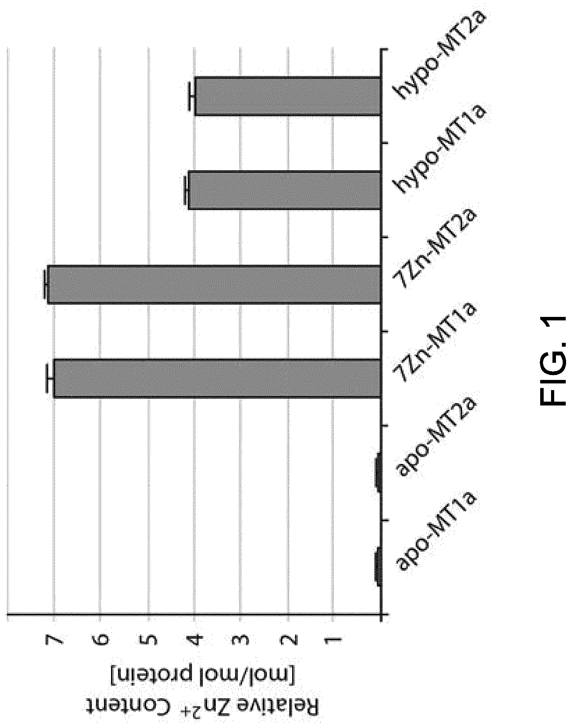

As shown in FIG.1, relative to the metal-free apo-proteins ("apo-MT1a" and "apo-MT2a") and the fully metallated controls (which bind 7 molar equivalents of Zn²⁺) ("7Zn-MT1a" and "7Zn-MT2a"), both hypoMT samples ("hypo-MT1a" and "hypo-MT2a") bind specific amounts of metal ions as defined by the reconstitution process (4 molar equivalents of Zn²⁺).

### 3. Hypo-MTs contain free reduced cysteine sulfhydryl groups

To investigate whether the free cysteine sulfur residues in hypo-MTs, as produced according to the invention, are present in chemically oxidized or reduced form, the zinc binding and releasing properties of said proteins were analyzed using the chromophoric zinc chelating agent Zincon (zincon monosodium salt, Sigma-Aldrich, MO, USA) according to the manufacturers protocol. In brief, equimolar amounts of MT proteins as well as controls were pre-incubated with 3 molar equivalents of zinc chloride in standard Hepes buffer at pH7.4, and then mixed with excess of Zincon reagent (100 µM Zincon per 1 µM of MT protein). Fluorescence signal was detected at 620nm with a Beckman Coulter DU800 spectrophotometer. Results from five independent experiments are shown, standard deviations are included into the graphs. As "oxidation" controls, aliquots of the apo-proteins were chemically oxidized at their cysteine sulfur residues by pre-incubation with 500 µM H₂O₂ prior to use in this assay ("OxApo-MT1a" and "OxApo-MT2a"). A blank sample with all buffers and reagents including zinc chloride, but without any MT protein, was included for normalization.

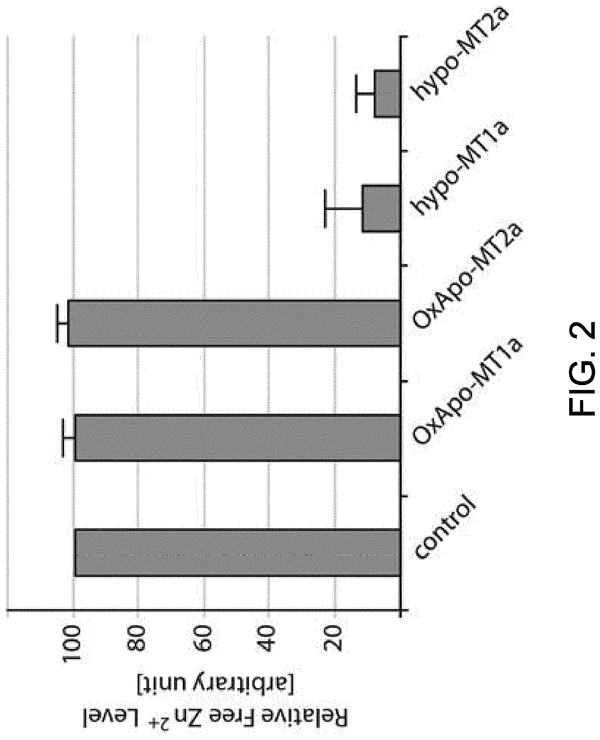

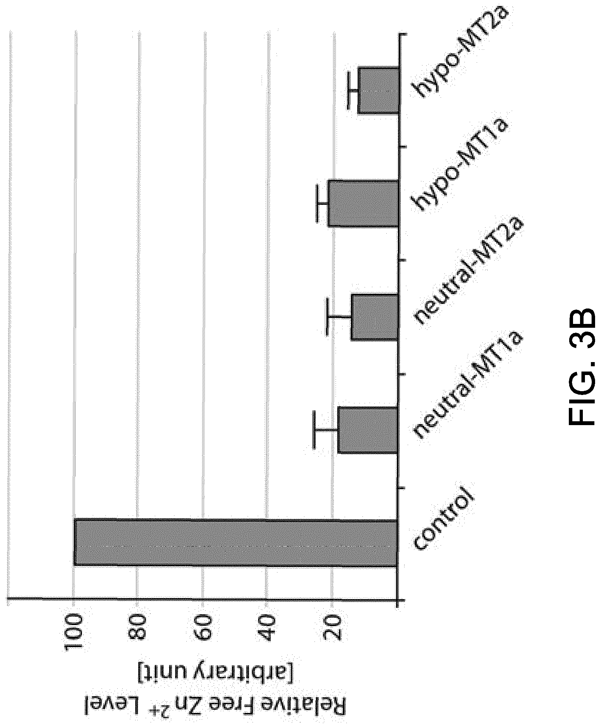

As shown in FIG. 2, oxidized cysteine sulfur residues as in the "oxidation" controls ("OxApo-MT1a" and "OxApo-MT2a"), are no longer able to coordinately bind zinc ions (indicated by same free zinc levels as in blank control). In contrast, both hypo-MT isoforms ("hypo-MT1a" and "hypo-MT2a") are able to bind three more molar equivalents of zinc ions (and thereby lower the levels of free zinc), indicating their free cysteine sulfurs being chemically reduced (see also FIG. 3B).

### 4. Hypo-MTs can redox-dependently bind or release metal ions

The above-mentioned chromophoric Zincon assay was also used to determine the ability of hypo-MT proteins, as per the invention, to bind or release zinc ions in response to changes in the redox potential. To measure potential zinc release under oxidative conditions, equimolar amounts of hypo-MT proteins as well as controls were mixed with excess of Zincon reagent (100 µM Zincon per 1 µM of MT protein), in pH7.4 Hepes buffer. After addition of 250 µmolar H₂O₂, fluorescence was detected at 620nm. To measure potential zinc binding under reductive conditions, equimolar amounts of hypo-MT proteins were mixed with 750 µmolar DTT, and subsequently with three molar equivalents of zinc chloride, in Hepes buffer at pH 7.4. After addition of excess of Zincon reagent (100 µM Zincon per 1 µM of MT protein), in pH7.4 Hepes buffer, fluorescence was detected at 620nm. For both assays, results from six independent experiments are shown, inclusive standard deviation. Blank samples with all buffers and reagents (inclusive 4 molar equivalents of zinc chloride for the oxidation assay, and 3 molar equivalents of zinc chloride for the reduction assay), but without any MT proteins, were included for normalization. "Neutral" controls of the reduction assay were treated identically, but without addition of DTT ("neutral-MT1a" and "neutral-MT2a").

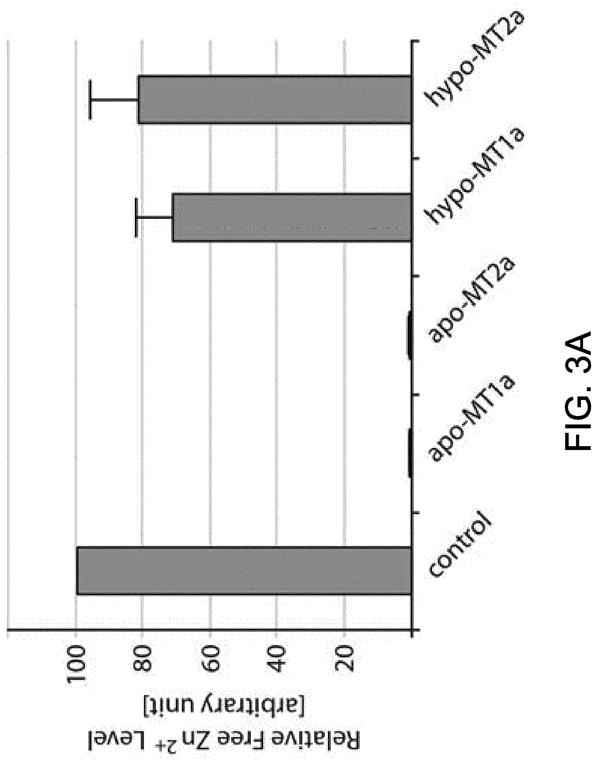

As shown in FIG. 3A, in contrast to the apo-proteins ("apo-MT1a" and "apo-MT2a") as negative controls, both hypo-MT proteins ("hypo-MT1a" and "hypo-MT2a") are able to release zinc ions under oxidative conditions.

As shown in FIG. 3B, both hypo-MT proteins ("hypo-MT1a" and "hypo-MT2a") are able to bind zinc ions under reductive conditions. Also, relative to the "neutral" controls ("neutral-MT1a" and "neutral-MT2a"), no increase in zinc binding capacity is seen upon reduction with DTT, further validating the above results of free cysteine sulfur residues of hypo-MT proteins already being in reduced form.

### 5. Hypo-MTs can balance redox potential and protectfrom oxidative stress

To test for capability of hypo-MT proteins, produced according to the invention, of protecting against oxidative stress and of balancing intracellular redox potential, the metabolic integrity of mouse liver cells was assayed under oxidative stress conditions, using the non-radioactive cell proliferation kit from Promega (Promega, WI, USA) according to the manufacturers protocols.

In brief, wild type mouse hepatocytes were taken into primary culture by a standard hepatic portal vein perfusion method (Butler, M. Animal Cell Culture and Technology: The Basics. 2nd Edition, 2003, Garland Publishing Inc.), and grown in 96 well microtiter plates in standard Dulbecco's Eagle medium to equal density of approximately 10,000 cells per well. Hypo-MT proteins were added to the respective samples, at 10 µM/ml, 24 hours prior to the assay. After exchanging the medium to clear out external hypo-MT proteins, H₂O₂ was added for 6 hours at 250 µM as oxidative stressor, and metabolic integrity was assayed by measuring the formation of "formazan" from "MTS inner salt" (Promega Cell Proliferation Kit), at 490nm using a BioTek FLx800TB Microplate reader. Experiments were repeated five times, and results were normalized to a blank control (minus H₂O₂ or hypo-MT proteins). Standard deviations are included in the graphs. Statistical analysis was done by paired Student's t-test, with *** indicating p<0.0001.

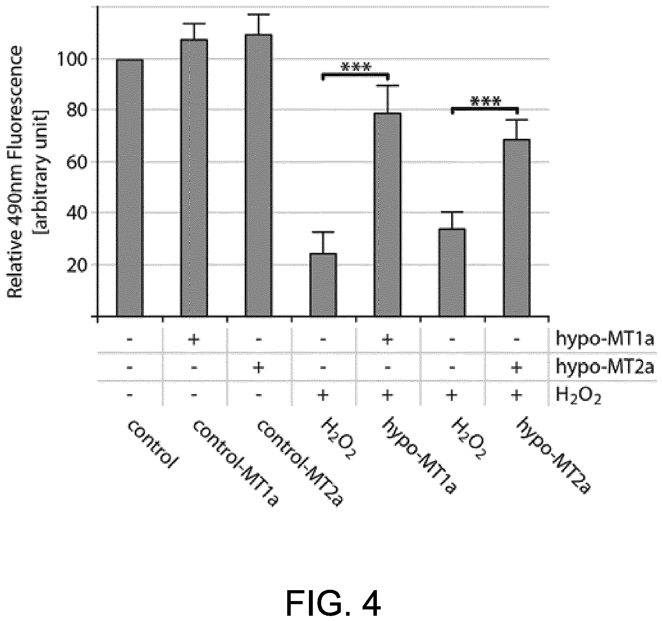

As shown in FIG. 4, relative to the controls, both hypo-MT variants of the invention can significantly protect from oxidative stress, by balancing intracellular redox potential and stabilizing metabolic integrity.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments are chosen and described in order to explain the principles of the invention and their practical application so as to activate others skilled in the art to utilize the invention and various embodiments and with various modifications as are suited to the particular use contemplated. Alternative embodiments will become apparent to those skilled in the art. Accordingly, to promote the progress in science and the useful arts, the scope of the present invention is disclosed and is intended to be limited only defined by the scope of the appended claims rather than the foregoing description and the exemplary embodiments described therein.

## Claims

1. A method for producing a hypo-metallated redox-active metallothionein protein, comprising the following steps:
providing a metallothionein protein sharing the amino acid composition and structural motifs conserved in all known naturally occurring mammalian metallothioneins, wherein such proteins contain exactly 20 cysteine residues at conserved positions, forming exactly seven redox-active binding pockets, each of which allows for coordinated binding of one divalent metal ion;
de-metallating the metallothionein protein;
chemically reducing all the 20 cysteine sulfur residues of the de-metallated metallothionein protein to the chemically reduced sulfhydryl (-SH) form; and
partially metallating the reduced de-metallated metallothionein protein by providing 1, 2, 3, 4, 5, or 6 metal ions to the 7 binding pockets;
wherein the as-produced hypo-metallated redox-active metallothionein protein has 20 cysteine sulfhydryl groups and 7 binding pockets, wherein 2 to 16 of the 20 cysteine sulfhydryl groups are free and reduced, and wherein 1 to 6 of the 7 binding pockets are occupied by metal ions.

2. The method of claim 1, wherein the metallothionein protein has an amino acid formula of
[Xₙ₁CXCXₙ₂CXCXₙ₃CXCXₙ₄CXCXₙ₅CXₙ₆CXₙ₇CXₙ₈CXₙ₉CXCXₙ₁₀CXCCXₙ₁₁],
wherein
X is any biogenic L-amino acid with the exception of cysteine, phenylalanine, tryptophan, and tyrosine; and
n1 to n11 are integers of any value from 1 to 12.

3. The method of claim 1, further comprising a step of fully pre-metallating the metallothionein protein before the step of de-metallating the metallothionein protein.

4. The method of claim 1, wherein the step of de-metallating the metallothionein protein is performed by acidifying the metallothionein protein to a pH value below pH 4.

5. The method of claim 1, further comprising a step of purifying the de-metallated metallothionein protein before the step of chemically reducing the de-metallated metallothionein protein.

6. The method of claim 5, wherein the step of purifying the de-metallated metallothionein protein is performed by one of dialysis, cation-exchange chromatography, reverse-phase chromatography, high-pressure-liquid chromatography, electrophoretic methods, gel filtration, and magnetic force.

7. The method of claim 1, wherein the step of chemically reducing the de-metallated metallothionein protein is performed by adding a reducing agent.

8. The method of claim 7, wherein the reducing agent is one of glutathione, reduced nicotinamide adenine dinucleotide (NADH), nicotinic acid, and dithiothreitol (DTT).

9. The method of claim 1, wherein the metal ions are selected from the group consisting of zinc, copper, iron, and selenium.

## Patentansprüche

1. Verfahren zur Herstellung von hypo-metalliertem redoxaktivem Metallothionein - Protein, umfassend die folgenden Schritte:
Bereitstellen eines Metallothionein-Proteins, das die Aminosäurezusammensetzung und die Strukturmotive teilt, die in allen bekannten natürlich vorkommenden Säugetier-Metallothioneinen konserviert sind,
wobei solche Proteine genau 20 Cysteinreste an konservierten Stellen enthalten, die genau sieben redoxaktive Bindungsstellen bilden, von denen jede die koordinierte Bindung eines zweiwertigen Metallions ermöglicht,
Entmetallieren des Metallothionein-Proteins,
chemische Reduktion aller 20 Cystein-Schwefel-Reste des entmetallierten Metallothionein-Proteins in die chemisch reduzierte Sulfhydrylform (-SH) und teilweises Metallieren des reduzierten entmetallierten Metallothionein-Proteins durch Bereitstellen von 1, 2, 3, 4, 5 oder 6 Metallionen an den 7 Bindungsstellen, wobei das hergestellte hypo-metallierte redoxaktive Metallothionein-Protein 20 Cysteinsulfhydrylgruppen und 7 Bindungsstellen aufweist, wobei 2 bis 16 der 20 Cysteinsulfhydrylgruppen frei und reduziert sind, und wobei 1 bis 6 der Bindungsstellen mit Metallionen besetzt sind.

2. Verfahren nach Anspruch 1, wobei das Metallothionein-Protein eine Aminosäuresequenz aus
[Xₙ₁CXCXₙ₂CXCXₙ₃CXCXₙ₄CXCXₙ₅CXₙ₆CCXCCXₙ₇CXₙ₈CXₙ₉CXCXₙ₁₀CXCCXₙ₁₁]
aufweist, wobei
X jede biogene L-Aminosäure mit Ausnahme von Cystein, Phenylalanin, Tryptophan und Tyrosin ist, und
n1 bis n11 ganze Zahlen von 1 bis 12.

3. Verfahren nach Anspruch 1 umfasst weiterhin einen Schritt des vollständigen Vormetallierens des Metallothionein-Proteins vor dem Schritt des Entmetallierens des Metallothionein-Proteins.

4. Verfahren nach Anspruch 1, bei welchem der Schritt des Entmetallierens des Metallothionein-Proteins durch Ansäuern des Metallothionein-Proteins auf einen pH-Wert unter 4 durchgeführt wird.

5. Verfahren nach Anspruch 1, welches weiterhin einen Schritt zur Reinigung des entmetallierten Metallothionein-Proteins vor dem Schritt der chemischen Reduktion des entmetallierten Metallothionein-Proteins umfasst.

6. Verfahren nach Anspruch 5, bei welchem der Schritt zur Reinigung des entmetallierten Metallothionein-Proteins durch Dialyse, Kationenaustausch-Chromatographie, Umkehrphasen-Chromatographie, Hochdruckflüssigkeitschromatographie, elektrophoretische Verfahren, Gel-Filtration und Magnetkraft durchgeführt wird.

7. Verfahren nach Anspruch 1, bei welchem der Schritt der chemischen Reduktion des entmetallierten Metallothionein-Proteins durch Hinzufügen eines Reduktionsmittels durchgeführt wird.

8. Verfahren nach Anspruch 7, bei welchem das Reduktionsmittel eines von Glutathion, reduziertes Nicotinamidadenindinukleotid (NADH), Nikotinsäure und Dithiothreitol (DTT) ist.

9. Verfahren nach Anspruch 1, bei welchem die Metallionen aus der Gruppe bestehend aus Zink, Kupfer, Eisen und Selen ausgewählt werden.

## Revendications

1. Procédé de production d'une protéine métallothionéine ayant subi une hypométallation et à activité redox, comprenant les étapes suivantes:
la fourniture d'une protéine métallothionéine partageant la composition et les motifs structuraux de l'acide aminé conservé dans toutes les métallothionéines de mammifère connues d'origine naturelle, dans lequel de telles protéines contiennent exactement 20 résidus cystéine à des positions conservées, formant exactement sept poches de liaison à activité redox, dont chacune permet la liaison coordonnée d'un ion de métal divalent;
la démétallation de la protéine métallothionéine;
la réduction chimique de la totalité des 20 résidus de soufre de cystéine de la protéine métallothionéine ayant subi une démétallation en la forme sulfhydryle (-SH) chimiquement réduite; et
la métallation partielle de la protéine métallothionéine ayant subi une démétallation réduite en fournissant 1, 2, 3, 4, 5, ou 6 ions de métal aux 7 poches de liaison;
dans lequel la protéine métallothionéine ayant subi une hypométallation et à activité redox telle que produite comporte 20 groupes de sulfhydryle de cystéine et 7 poches de liaison, dans lequel 2 à 16 des 20 groupes de sulfhydryle de cystéine sont libres et réduits, et dans lequel 1 à 6 des 7 poches de liaison sont occupées par des ions de métal.

2. Procédé selon la revendication 1, dans lequel la protéine métallothionéine a une formule d'acide aminé de
[Xₙ₁CXCXₙ₂CXCXₙ₃CXCXₙ₄CXCXₙ₅CXₙ₆CCXCCXₙ₇CXₙ₈CXₙ₉CXCXₙ₁₀CXCCXₙ₁₁]
, dans laquelle
X est un acide L-aminé biogène quelconque à l'exception de la cystéine, de la phénylalanine, du tryptophane, et de la tyrosine; et
n1 à n11 sont des entiers d'une valeur quelconque de 1 à 12.

3. Procédé selon la revendication 1, comprenant en outre une étape de pré-métallation complète de la protéine métallothionéine avant l'étape de démétallation de la protéine métallothionéine.

4. Procédé selon la revendication 1, dans lequel l'étape de démétallation de la protéine métallothionéine est réalisée par acidification de la protéine métallothionéine à une valeur de pH en dessous de pH 4.

5. Procédé selon la revendication 1, comprenant en outre une étape de purification de la protéine métallothionéine ayant subi une démétallation avant l'étape de réduction chimique de la protéine métallothionéine ayant subi une démétallation.

6. Procédé selon la revendication 5, dans lequel l'étape de purification de la protéine métallothionéine ayant subi une démétallation est réalisée par l'un quelconque parmi une dialyse, une chromatographie échangeuse de cations, une chromatographie en phase inverse, une chromatographie liquide haute pression, des procédés électrophorétiques, une filtration sur gel, et une force magnétique.

7. Procédé selon la revendication 1, dans lequel l'étape de réduction chimique de la protéine métallothionéine ayant subi une démétallation est réalisée par ajout d'un agent de réduction.

8. Procédé selon la revendication 7, dans lequel l'agent de réduction est l'un parmi la glutathione, le nicotinamide adénine dinucléotide (NADH) réduit, l'acide nicotinique, et le dithiothréitol (DTT).

9. Procédé selon la revendication 1, dans lequel les ions de métal sont choisis dans le groupe constitué du zinc, du cuivre, du fer, et du sélénium.


### SEQUENCE LISTING

<110> Melcher, Christoph
   Henry, Tony Raoul
   Kuo, Chun-Hung
<120> Method For Producing Hypo-Metallated Redox-Active Metallothionein Protein And Pharmaceutical Composition Containing The Same
<130> P14-109
<160> 4
<170> PatentIn version 3.5
<210> 1
   <211> 186
   <212> DNA
   <213> Homo sapiens
<300>
   <308> GenBank NM_005946
   <309> 2014-01-19
   <313> (74)..(259)
<400> 1
   atggacccca actgctcctg cgccactggt ggctcctgca cctgcactgg ctcctgcaaa      60
   tgcaaagagt gcaaatgcac ctcctgcaag aagagctgct gctcctgctg ccccatgagc     120
   tgtgccaagt gtgcccaggg ctgcatctgc aaaggggcat cagagaagtg cagctgctgt     180
   gcctga                                                                186
<210> 2
   <211> 61
   <212> PRT
   <213> Homo sapiens
<300>
   <308> GenBank NP_005937
   <309> 2014-01-19
   <313> (1)..(61)
<400> 2
   Met Asp Pro Asn Cys Ser Cys Ala Thr Gly Gly Ser Cys Thr Cys Thr
   1               5                   10                  15
   Gly Ser Cys Lys Cys Lys Glu Cys Lys Cys Thr Ser Cys Lys Lys Ser
               20                  25                  30
   Cys Cys Ser Cys Cys Pro Met Ser Cys Ala Lys Cys Ala Gln Gly Cys
           35                  40                  45
   Ile Cys Lys Gly Ala Ser Glu Lys Cys Ser Cys Cys Ala
       50                  55                  60
<210> 3
   <211> 186
   <212> DNA
   <213> Bos taurus
<300>
   <308> GenBank NM_001075140
   <309> 2014-02-08
   <313> (79)..(264)
<400> 3
   atggatccca actgctcctg caccgcgggt gaatcctgca cgtgtgccgg ctcctgcaaa      60
   tgcaaagatt gcaagtgcgc ctcctgcaag aagagctgct gctcctgctg ccccgtgggc     120
   tgtgccaagt gtgcccaggg ctgcgtctgc aaaggggctt cggacaagtg cagctgctgt     180
   gcctga                                                                 186
<210> 4
   <211> 61
   <212> PRT
   <213> Bos taurus
<300>
   <308> GenBank NP_001068608
   <309> 2014-02-08
   <313> (1)..(61)
<400> 4
   Met Asp Pro Asn Cys Ser Cys Thr Ala Gly Glu Ser Cys Thr Cys Ala
   1               5                   10                  15
   Gly Ser Cys Lys Cys Lys Asp Cys Lys Cys Ala Ser Cys Lys Lys Ser
               20                  25                  30
   Cys Cys Ser Cys Cys Pro Val Gly Cys Ala Lys Cys Ala Gln Gly Cys
           35                  40                  45
   Val Cys Lys Gly Ala Ser Asp Lys Cys Ser Cys Cys Ala
       50                  55                  60